# FASCICULE DE BREVET EUROPEEN

(11) **EP 3 397 265 B1**
(45) Date de publication et mention de la délivrance du brevet: **29.07.2020**
(21) Numéro de dépôt: 16829300.9
(22) Date de dépôt: 30.12.2016
(51) Int. Cl.: A61K 38/17, A23L 33/00, A23L 33/19, A23L 2/66, A61K 35/20, A61P 3/00

(54) **COMPOSITION DE NUTRITION ENTERALE RICHE EN PROTEINES A FORTE PROPORTION DE CASEINATES**
PROTEINREICHE ENTERALE NÄHRSTOFFZUSAMMENSETZUNG MIT EINEM HOHEN ANTEIL VON KASEINATEN
PROTEIN-RICH ENTERAL NUTRITIONAL COMPOSITION CONTAINING A HIGH PROPORTION OF CASEINATES

(30) Priorité: 30.12.2015 FR 1563471
(43) Date de publication de la demande: 07.11.2018
(73) Titulaire: Even Santé Industrie, 29260 Lesneven (FR)
(72) Inventeur: LE FUR, Audrey, 29860 Bourg Blanc (FR); LE PALUD, Jean-Marc, 29860 Plabennec (FR); FOSSEUX, Anne, 29200 Brest (FR)
(74) Mandataire: IP Trust
(86) Numéro de dépôt international: PCT/FR2016/053688
(87) Numéro de publication internationale: WO 2017/115058

(56) Documents cités:
- EP-A1- 2 230 940
- WO-A1-2009/072869

## Description

### DOMAINE DE L'INVENTION

La présente invention se rapporte au domaine des compositions nutritionnelles à des fins médicales spéciales à forte densité énergétique et riches en protéines et à leur procédé de préparation. Ces compositions nutritionnelles sont à destination des personnes âgées, des personnes malades, affaiblies ou en état de dénutrition liée à la maladie. Ces compositions sont utilisables en source principale d'alimentation ou en complément.

La présente invention porte plus précisément sur une composition nutritionnelle buvable hyperénergétique et riche en protéine, particulièrement riche en caséinates, stable dans le temps, unidose et prête à l'emploi et son procédé de préparation. La composition selon l'invention est destinée à des adultes et peut être utilisée en source principale d'alimentation ou en complément.

### CONTEXTE GENERAL DE L'INVENTION

Le corps humain est un organisme complexe. Le maintien des fonctions vitales nécessite de l'énergie qui est apportée par l'alimentation.

L'alimentation apporte de l'énergie quantifiable sous forme de kilojoules ou de kilocalories.

Le bon fonctionnement du corps nécessite une alimentation suffisante et équilibrée. L'énergie doit être apportée par des protéines, des sucres et des matières grasses. Les protéines sont indispensables à l'activité musculaire, les matières grasses permettent de stocker de l'énergie libérable en fonction des besoins du corps, et les sucres apportent une source immédiatement disponible d'énergie. Ces sources caloriques sont donc complémentaires. Elles doivent être complétées par des apports en vitamines et minéraux qui interviennent dans l'équilibre physiologique des différentes fonctions vitales du corps.

Cet équilibre peut être rompu en cas de malnutrition due par exemple à un trouble de l'alimentation (anorexie...), ou de dénutrition conséquente à une situation pathologique ou chez les personnes âgées qui ne s'alimentent plus correctement de façon autonome.

Les besoins nutritionnels évoluent au cours de la vie. Par exemple, les personnes âgées ont des besoins réduits en lipides, dus à une diminution d'activité et à un ralentissement du renouvellement cellulaire.

Certaines personnes, par exemple à cause de la maladie, ne sont plus capables de s'alimenter correctement, voire même de s'alimenter complètement. Il est donc nécessaire de mettre en place une alimentation de supplémentation ou de substitution afin de subvenir à leurs besoins nutritionnels.

Lorsque le tube digestif est toujours fonctionnel, la nutrition entérale, c'est à dire *per os* ou par sonde, est privilégiée. Elle peut se faire avec des compositions nutritionnelles adaptées en termes de profil nutritionnel, de viscosité ou encore de volume.

A titre d'exemple, certains patients ne peuvent pas ingérer de volumes importants d'aliments. Il s'agit par exemple des patients cachectiques en lien ou non avec une pathologie, par exemple le syndrome d'immunodéficience acquise, un cancer, des maladies respiratoires ou infectieuses, ou des traumatismes.

### ETAT DE LA TECHNIQUE

Il est connu de l'art antérieur des compositions de nutrition entérale riches en protéines et hyperénergétiques.

La demande de brevet WO2014/099795 décrit des compositions nutritives orales à faible viscosité et à haute densité calorique. Les compositions de cette demande comprennent majoritairement des protéines de lait non micellaires sous forme de poudre, en association avec du caséinate hydrolysé.

Le brevet EP2230940 décrit une composition de nutrition entérale liquide à haute densité énergétique comprenant de la caséine micellaire, des caséinates et optionnellement une faible quantité de petit lait.

La demande de brevet WO2002/098242 propose un complément liquide buvable hautement calorique dont les protéines totales sont constituées uniquement de caséinates ou d'un mélange de caséinates et de protéines de soja.

### Inconvénients de l'art antérieur

Les solutions de l'art antérieur utilisent des protéines qui peuvent avoir perdu leur forme native. Les compositions de l'art antérieur dont les protéines totales consistent en des protéines de lait comprennent généralement une majorité de caséines. Or les caséines peuvent réagir avec les acides et autres ingrédients présents dans la composition de nutrition. Ces réactions entraînent la formation de sels de calcium dans le temps résultant en une faible stabilité des produits après conditionnement.

Des solutions de l'état de la technique ont consisté à remplacer ces caséines par des caséinates ou à mélanger ces caséines à des caséinates, les caséinates étant dans ce cas minoritaires par rapport aux caséines. Les caséinates sont plus résistants à la chaleur, mais ils augmentent la viscosité de la composition nutritionnelle, pouvant la rendre plus difficile à consommer par les patients cibles ne pouvant ingérer des compositions trop visqueuses.

Enfin, les compositions nutritionnelles riches en protéines et en énergie présentent souvent des problèmes organoleptiques qui les rendent non appétissantes pour les patients. Or, les patients susceptibles de consommer ces produits nutritionnels ont peu d'appétit, il est donc primordial de favoriser la préservation de bonnes propriétés organoleptiques et une forte appétence.

Il existe un réel besoin de formuler des compositions de nutrition entérale prêtes à l'emploi ayant des propriétés organoleptiques améliorées, stables, appétissantes, tout en restant suffisamment liquides pour être facilement bues par les patients.

### SOLUTION APPORTEE PAR L'INVENTION

La présente invention se propose donc de remédier aux inconvénients de l'art antérieur par la fourniture d'une composition de nutrition entérale buvable, appétissante, stable, aux propriétés organoleptiques améliorées favorisant la prise par des patients à l'appétit diminué. La composition de la présente invention apporte une quantité augmentée de calories et de protéines tout en étant suffisamment fluide pour être bue au verre ou à la paille.

La composition de nutrition entérale est thermostérilisée et prête à l'emploi.

La présente invention porte donc sur une composition de nutrition entérale comportant par 100 mL de composition :
- 8 à 14 g de protéines totales
- 837 à 1465 kJ (200 à 350 kcal)
- les protéines totales comportant :
   ∘ une quantité de caséinates supérieure ou égale à 55% en poids desdites protéines totales de la composition
   ∘ une quantité de protéines sériques inférieure ou égale à 30% en poids desdites protéines totales de la composition
   ∘ une quantité de caséines inférieure ou égale à 30% en poids desdites protéines totales de la composition,
le ratio en poids desdits caséinates auxdites caséines étant supérieur ou égal à 70/30.

L'on comprend que les protéines totales de la composition de nutrition entérale selon l'invention sont majoritairement constituées de caséinates. Le ratio en poids des caséinates aux caséines est supérieur à 70/30. L'on comprend que la proportion de caséinates par rapport aux caséines est supérieure ou égale à 70% du poids combiné des caséinates et des caséines.

Toutefois, en augmentant la proportion de caséinates, les inventeurs ont eu à maitriser l'augmentation de la viscosité pour que la composition reste buvable. Pour ce faire, ils ont mis au point un procédé de préparation permettant d'obtenir une composition thermostérilisée présentant une forte proportion en caséinates, en particulier un ratio en poids des caséinates aux caséines supérieur ou égal à 70/30, mais dont la viscosité reste inférieure ou égale à 800 mPa.s, de préférence inférieure à 450 mPa.s quand mesurée à 20°C à 100 s⁻¹ avec un viscosimètre rotatif. Ce procédé comprend une étape de stérilisation réalisée selon les procédés habituels d'Ultra Haute Température, et dont la valeur stérilisatrice (f0) résultante est supérieure ou égale à 12.

Grace à ce procédé, la demanderesse a obtenu de façon surprenante que ce mélange particulier de protéines permettait d'obtenir une composition de nutrition entérale riche en caséinates qui est buvable, appétissante, stable, aux propriétés organoleptiques améliorées favorisant la prise par des patients à l'appétit diminué.

Avantageusement, les caséinates sont choisis parmi les caséinates de sodium, les caséinates de potassium, les caséinates de calcium ou un mélange d'au moins deux parmi ceux-ci.

Dans un mode de réalisation, la quantité de protéines sériques est inférieure ou égale à 25% en poids des protéines totales de la composition.

Dans un mode de réalisation, la quantité de caséine est inférieure ou égale à 25% en poids des protéines totales de la composition.

Avantageusement, les protéines contribuent pour 10 à 40% des apports énergétiques totaux par 100 mL de composition.

La viscosité de la composition après thermostérilisation est inférieure ou égale à 800 mPa.s quand mesurée à 20°C à 100 s⁻¹ avec un viscosimètre rotatif.

Dans un mode de réalisation préféré, la viscosité de la composition après thermostérilisation est comprise entre 30 et 450 mPa.s quand mesurée à 20°C à 100 s⁻¹ avec un viscosimètre rotatif.

Avantageusement, la composition de nutrition entérale est utilisée pour nourrir une personne âgée, une personne dénutrie, une personne en convalescence ou atteinte de maladie chronique.

### DESCRIPTION DETAILLEE DE L'INVENTION

Un premier objet de l'invention consiste en une composition de nutrition entérale thermostérilisée comportant par 100 mL de composition :
- 8 à 14 g de protéines totales
- 837 à 1465 kJ (200 à 350 kcal)
- les protéines totales comportant :
   ∘ une quantité de caséinates supérieure ou égale à 55% en poids desdites protéines totales de la composition
   ∘ une quantité de protéines sériques inférieure ou égale à 30% en poids desdites protéines totales de la composition
   ∘ une quantité de caséines inférieure ou égale à 30% en poids desdites protéines totales de la composition,
dans laquelle le ratio en poids desdits caséinates auxdites caséines étant supérieur ou égal à 70/30,
et pour laquelle la viscosité est inférieure ou égale à 800 mPa.s quand mesurée à 20°C à 100 s⁻¹ avec un viscosimètre rotatif.

La composition selon l'invention peut se présenter sous différentes formes en fonction des besoins des patients auxquels elle est destinée.

Dans un mode de réalisation particulier, la composition de nutrition entérale comprend un ratio en poids de caséinates/caséines/protéines sériques d'environ 60/25/15.

Selon un mode de réalisation préféré, la composition de nutrition entérale comprend :
- 9 à 12 g de protéines totales par 100 mL de composition
- une densité énergétique d'environ 1046 kJ (250 kcal) par 100 mL de composition
- un ratio en poids de caséinates/caséines/protéines sériques d'environ 60/25/15.

Selon un autre mode de réalisation, la composition de nutrition entérale comprend 8 à 14 g de protéines totales par 100 mL de composition.

Selon encore un mode de réalisation, la composition de nutrition entérale comprend 8 à 12 g de protéines totales par 100 mL de composition.

### Définitions :

Par « buvable » selon la présente invention, il est compris que la composition nutritionnelle est suffisamment fluide pour être bue dans un verre ou à la paille. Elle ne nécessite pas une administration à la cuillère. La composition de la présente invention n'est pas destinée à une administration par sonde. La viscosité d'une composition « buvable » selon la présente invention est inférieure ou égale à 800 mPa.s quand mesurée à 100 s⁻¹ à 20°C avec un viscosimètre rotatif. Le viscosimètre rotatif est du type cup and bob, par exemple un DIN coaxial cylinder. Préférentiellement, la viscosité est comprise entre 30 et 600 mPa.s, plus préférentiellement entre 30 et 450 mPa.s.

Par « thermostérilisée », il est compris que la composition avant son conditionnement est stérilisée par les techniques bien connues de l'Homme du métier comme la pasteurisation et/ou la stérilisation. Préférentiellement, la composition est thermostérilisée avant conditionnement aseptique.

La stérilisation peut être réalisée par un procédé à Ultra Haute Température (U.H.T.) classique et bien connu de l'homme du métier. Dans un mode de réalisation préféré, la stérilisation est réalisée par un procédé UHT dont la valeur stérilisatrice (f0) résultante est supérieure ou égale à 12.

Par « prête à l'emploi », il est compris que la composition thermostérilisée est conditionnée en bouteille, pack, briquette ou tout autre emballage. La composition est conditionnée en format unidose, généralement de 50, 100, 125, 150, 200, 250 ou 300 mL, préférentiellement de 125 mL. La composition ainsi conditionnée est de longue conservation, c'est-à-dire que la durée limite d'utilisation optimale (DLUO) est d'au moins 6 mois, préférentiellement d'au moins 9 mois, encore plus préférentiellement d'au moins 12 mois.

Lorsque des intervalles de valeurs sont définis par une borne basse et une borne haute, l'on comprend que les valeurs des bornes sont comprises dans lesdits intervalles.

### Protéines :

La composition nutritionnelle entérale buvable comprend entre 8 et 18 grammes de protéines par 100 mL de composition. Préférentiellement, la composition comprend entre 9 et 16 g de protéines par 100 mL, encore plus préférentiellement entre 9 et 14 g de protéines par 100 mL de composition. De manière tout à fait préférée, la composition nutritionelle entérale selon l'invention comprend entre 8 et 12g ou entre 9 et 12g de protéines par 100 mL de composition. La protéine comprend au moins une source de caséinates, au moins une source de caséines, au moins une source de protéines sériques (petit-lait). Dans un mode de réalisation, la composition comprend uniquement une source protéique laitière, apportée par du lait de vache ou ses dérivés.

Les caséinates sont apportés par des caséinates de sodium, des caséinates de potassium, des caséinates de calcium, ou un mélange d'au moins deux parmi ceux-ci.

La caséine est apportée par différents ingrédients que sont non limitativement des isolats de protéines de lait (MPI pour Milk Protein Isolate), des concentrés de protéines de lait (MPC pour Milk Protein Concentrate), des isolats de caséines micellaires (MCI), des protéines de lait concentré liquide, du lait écrémé, du lait écrémé concentré, ou un mélange d'au moins deux parmi ceux-ci. L'on comprend par caséine, la protéine constitutive de la micelle de caséine du lait. La caséine peut être sous forme micellaire ou non micellaire.

La composition nutritionnelle entérale buvable comprend une quantité de protéines sériques inférieure ou égale à 30% en poids des protéines totales de la composition. Les protéines sériques, c'est à dire les protéines de petit-lait, peuvent être apportées par les protéines totales de lait (sources de caséines), par des isolats de protéines sériques, des concentrés de protéines sériques ou un mélange d'au moins deux parmi ceux-ci. Préférentiellement, la composition comprend moins de 25% de protéines sériques par rapport au poids des protéines totales de la composition, encore plus préférentiellement moins de 20%. Dans un mode de réalisation, la composition comprend environ 15% de protéines sériques par rapport aux protéines totales de la composition.

Dans un mode de réalisation, la quantité combinée de caséinates et de caséines est d'au moins 70% en poids des protéines totales de la composition avec un ratio en poids des caséinates aux caséines supérieur ou égale à 70/30.

Dans un mode de réalisation, la quantité combinée de caséinates et de protéines sériques est d'au moins 60% en poids des protéines totales de la composition avec un ratio en poids des caséinates aux protéines sériques supérieur ou égal à 60/40.

Dans un mode particulier de réalisation, la quantité combinée de caséinates, de caséines et de protéines sériques est égale à 100% des protéines totales de la composition.

Dans un mode de réalisation, les protéines totales de la composition apportent entre 10 et 40% des apports énergétiques totaux (AET) de la composition. Dans un mode préféré de réalisation, les protéines apportent entre 12 et 35% des AET.

### Glucides :

La composition nutritionnelle entérale comprend en outre des glucides. Les glucides fournissent entre 15 et 50% des apports énergétiques totaux.

Les glucides peuvent être des glucides simples ou complexes ou un mélange parmi ceux-ci. Les glucides peuvent comprendre du glucose, du fructose, du sucrose, du lactose, du tréhalose, du palatinose, du sirop de maïs, du malt, du maltose, de l'isomaltose, de l'amidon de maïs partiellement hydrolysé, des maltodextrines, du sirop de glucose, du sucre, des oligosaccharides, des polysaccharides, des édulcorants ou un mélange parmi ceux-ci. Les glucides sont choisis pour limiter leur impact sur la viscosité de la composition nutritionnelle ainsi que pour éviter un goût sucré excessif, des réactions de Maillard trop importantes et une osmolarité trop élevée.

Dans un mode de réalisation pour une composition de nutrition entérale très fluide, le sirop de glucose sera privilégié. Dans un autre mode de réalisation pour une composition de nutrition entérale buvable mais moins fluide, les glucides pourront en partie être apportés par un sirop de glucose et/ou une maltodextrine.

Dans un mode particulier de réalisation, le sirop de glucose et/ou la maltodextrine peuvent être couplés à une autre source de glucides.

### Lipides :

La composition nutritionnelle entérale comporte en outre des lipides fournissant entre 20 et 65% des apports énergétiques totaux.

La composition selon l'invention comprend entre 8 et 30 g de lipides par 100 mL de composition.

Les lipides utilisés dans la composition sont des lipides alimentaires d'origines animales ou végétales. Dans un mode de réalisation de l'invention, les huiles végétales telles que les huiles de colza, de soja, de maïs ou de tournesol ou un mélange parmi celles-ci, sont préférées par leur plus faible teneur en cholestérol et/ou en acides gras saturés par rapport aux graisses d'origines animales.

La quantité combinée de lipides, glucides et protéines de la composition selon la présente invention est comprise entre 35 et 75 g par 100 mL de composition. Préférentiellement, la quantité combinée de lipides, glucides et protéines de la composition est comprise entre 45 et 65 g par 100 mL de composition.

### Les vitamines et minéraux :

La composition nutritionnelle selon l'invention comprend une grande variété de minéraux et de vitamines pour être au plus proche des apports journaliers recommandés. Dans un mode de réalisation de l'invention, la composition nutritionnelle présente un profil nutritionnel en termes de vitamines et de minéraux quasi complet à complet selon la réglementation européenne des aliments diététiques destinés à des fins médicales spéciales.

Les vitamines et minéraux sont majoritairement apportés au minimum à hauteur de 15% des apports journaliers recommandés définis par la législation européenne.

### Fibres :

Dans certains modes de réalisation, la composition peut être complémentée avec des fibres alimentaires. Ces fibres peuvent être des prébiotiques comme les fructo-oligosaccharides, les galacto-oligosaccharides ou l'inuline. La quantité de ces fibres peut être comprise entre 0,5 et 6 grammes par 100 mL de composition, préférentiellement entre 2 et 3 grammes par 100 mL de composition. Préférentiellement, les fibres alimentaires utilisées sont solubles. Elles peuvent être non limitativement des fructanes, des fructo-oligosaccharides, les galacto-oligosaccharides, des trans-galacto-oligosaccharides, des xylo-oligosaccharides, des arabino-oligosaccharides, des mano-oligosaccharides, des fuco-oligosaccharides, des oligosaccharides de soja, de l'inuline ou un mélange de ceux-ci. Des fibres insolubles telles que la cellulose et ses dérivés peuvent également être utilisées en complément des fibres solubles.

### Epaississants :

Pour répondre aux contraintes de pathologies particulières telles que la dysphagie, des épaississants peuvent être ajoutés à la composition de nutrition entérale pour augmenter sa viscosité.

Dans un mode particulier de réalisation, l'invention peut donc comprendre des épaississants comme, de l'amidon, des gommes végétales, des carraghénanes, de la caroube, du xanthane, du guar, des pectines, des alginates, de l'agar, des gélatines ou tout autre épaississant connu de l'Homme du métier.

Un second objet de l'invention consiste en un procédé de préparation d'une composition nutritionnelle entérale thermostérilisée telle que définie précédemment, en particulier une composition comprenant 8 à 14 g de protéines totales, dans laquelle le ratio en poids des caséinates aux caséines est supérieur ou égal à 70/30, et pour laquelle la viscosité est inférieure ou égale à 800 mPa.s quand mesurée à 20°C à 100 s⁻¹ avec un viscosimètre rotatif.

Un tel procédé comprend les étapes de :
- préparation d'une phase aqueuse,
- ajout dans ladite préparation aqueuse d'eau, de glucides, de protéines, de minéraux et de vitamines,
ajout de matière grasse,
- refroidissement et hydratation du mélange obtenu, et
- stérilisation de par un procédé U.H.T. dont la valeur stérilisatrice résultante est supérieure ou égale à 12,
une homogénéisation étant réalisée avant ou après l'étape de stérilisation pour stabiliser l'émulsion.

Dans des modes opératoires alternatifs, le procédé peut comprendre des étapes optionnelles telles que :
- l'ajout d'émulsifiants en même temps que l'ajout de la matière grasse, ou
- l'ajout de colorants et d'arômes.

De plus, au sens de l'invention, par « une valeur stérilisatrice à supérieure ou égale à 12 », il est compris un chambrage pouvant aller par exemple de 129°C pendant 2 minutes à 145°C pendant 5 secondes.

### EXEMPLES

L'invention sera mieux comprise à la lumière d'exemples non limitatifs de réalisation. Les présents exemples portent sur des boissons riches en protéines et hypercaloriques, nutritionnellement quasi complètes à complètes. Les compositions peuvent être utilisées comme seule source d'alimentation ou en complément. Ce sont des suppléments oraux appartenant à la catégorie des aliments diététiques destinés à des fins médicales spéciales, destinés à couvrir les besoins nutritionnels en cas de dénutrition liée à la maladie. Ils répondent à la législation en vigueur sur les produits de nutrition médicale.

### Exemple 1 : Procédé de préparation et conditionnement

La composition selon la présente invention peut être obtenue en préparant une phase aqueuse dans laquelle sont incorporés en une ou plusieurs étapes l'eau, les glucides, les protéines, les minéraux, les vitamines, les colorants, les arômes et les éventuels autres additifs. L'eau peut être préalablement chauffée entre 30 et 60°C. La matière grasse et les émulsifiants peuvent être directement ajoutés ou préparés à part (phase grasse comportant des huiles et/ou des émulsifiants chauffée et incorporée dans la phase aqueuse).

Une phase de refroidissement et d'hydratation est appliquée sur le mélange ainsi obtenu. La composition est ensuite stérilisée selon les procédés habituels d'Ultra Haute Température, avec un chambrage pouvant aller de 129°C pendant 2 minutes à 145°C pendant 5 secondes; la valeur stérilisatrice (f0) résultante étant >=12 .

Afin de stabiliser l'émulsion, une homogénéisation est pratiquée avant ou après stérilisation, soit durant la phase de montée en température (chauffage), soit durant le refroidissement avant conditionnement aseptique.

### Exemple 2 : Compositions

Trois compositions A, B et C sont présentées ci-dessous au Tableau 1. Elles sont aromatisées à la vanille, mais elles peuvent bien entendu, être aromatisées à n'importe quel autre parfum comme chocolat, café, fraise, framboise, citron...

Les compositions nutritionnelles sont ici stérilisées UHT (Ultra Haute Température) permettant d'avoir une valeur stérilisatrice de 12 minutes.

Les compositions sont prêtes à l'emploi et conditionnées dans des bouteilles adaptées de 125 mL, correspondant à une portion.

Ces compositions sont buvables à la paille ou au verre.

Le tableau 1 présente donc trois exemples de réalisation de l'invention. La composition A comprend 9 g de protéines par 100 mL de composition, la composition B comprend 10 g de protéines par 100 mL de composition et la composition C comprend 12 g de protéines par 100 mL de composition.

**Tableau 1. Compositions A, B et C selon l'invention**

| | ar 100 mL | | AET | | AET | | AET |
|---|---|---|---|---|---|---|---|
| Energie | cal | **50** | **00** | **52** | **00** | **51** | **00** |
| Protéines | | | 4 | **0** | 6 | **2** | 9 |
| *Caséine (% en poids des protéines totales)* | | *5,0%* | | *5,0%* | | *5,0%* | |
| *Caséinates (% en poids des protéines totales)* | | *0,0%* | | *0,0%* | | *0,0%* | |
| *Protéines sériques (% en poids des protéines totales)* | | *5,0%* | | *5,0%* | | *5,0%* | |
| *Caséines* / *caséinates* | | *0*/*70* | | *0*/*70* | | *0*/*70* | |
| Lipides | | **0,4** | 8 | **0,2** | 6 | **0,1** | 6 |
| *acides gras saturés* | | | | | | | |
| | | *,8* | | *,8* | | *,8* | |
| *acides gras moninsaturés* | | *,1* | | *,1* | | *,1* | |
| *acides gras polyinsaturés* | | *,9* | | *,9* | | *,9* | |
| Glucides | | **0** | 8 | **0** | 8 | **8** | 5 |
| *mono-disaccharides* | | *,1* | | *,1* | | *,1* | |
| Fibres | | | | | | | |
| Viscosité (à 20°C à 100 s⁻¹) | Pa.s | 50 | | 24 | | 33 | |

Le tableau 2 décrit de manière plus détaillée une composition D comprenant 10 g de protéines par mL de composition. Dans cette composition, les teneurs en vitamines sont ajustées pour couvrir les besoins nutritionnels selon la législation en vigueur

**Tableau 2 : Liste détaillée des composants d'une composition D**

| | |
|---|---|
| Taille de la portion | 125 mL |
| Densité | 1,145 g/mL |
| **Composition pour 100ml** | |
| **Energie** | **250 kcal** |
| **PROTEINES** | **10 g** |
| Caséine (% /protéines) | 24,0% |
| Caséinates (% des protéines) | 59,0% |
| Protéines sériques (% des protéines) | 17,0% |
| **Caséine / caséinate** | **29/71** |
| **LIPIDES** | **10 g** |
| acides gras saturés | 0,8 g |
| acides gras moninsaturés | 6,1 g |
| acides gras polyinsaturés | 2,9 g |
| acide linoleique | 2,03 g |
| w6/w3 | 2,3 g |
| **GLUCIDES** | **30 g** |
| mono-disaccharides | 6,2 g |
| polysaccharides | 24 g |
| saccharose | 1,5 g |
| lactose | < 0,5 g |
| **MINERAUX** | |
| Calcium | 150 mg |
| Copper | 350 mg |
| Iron | 1,5 mg |
| Potassium | 240 mg |
| Magnésium | 25 mg |
| Manganèse | 0,36 mg |
| Sodium | 95 mg |
| Phosphorus | 160 mg |
| Zinc | 1,6 mg |
| Chromium | 15 mg |
| Chloride | 100 mg |
| Fluor | 0,01 mg |
| Molybdène | 14 mg |
| Iodine | 4 mg |
| Sélénium | 14 mg |

Le tableau 3 décrit une composition E comprenant 10g de protéines par mL et dans laquelle le ratio caséinate/caséine est égal à 100/0. Les teneurs en vitamines sont ajustées pour couvrir les besoins nutritionnels selon la législation en vigueur.

**Tableau 3 : Liste détaillée des composants d'une composition E**

| | |
|---|---|
| Taille de la portion | 125 mL |
| Densité | 1,145 g/mL |
| **Composition pour 100ml** | |
| **Energie** | **250 kcal** |
| **PROTEINES** | **10 g** |
| Caséine (% /protéines) | 0 % |
| Caséinates (% des protéines) | 88% |
| Protéines sériques (% des protéines) | 12% |
| **Caséine / caséinate** | **0/100** |
| **LIPIDES** | **10 g** |
| acides gras saturés | 0,8 g |
| acides gras moninsaturés | 6,1 g |
| acides gras polyinsaturés | 2,9 g |
| acide linoleique | 2,03 g |
| w6/w3 | 2,3 g |
| **GLUCIDES** | **30 g** |
| mono-disaccharides | 6,2 g |
| polysaccharides | 24 g |
| saccharose | 1,5 g |
| lactose | < 0,5 g |
| **MINERAUX** | |
| Calcium | 92 mg |
| Copper | 350 mg |
| Iron | 1,5 mg |
| Potassium | 250 mg |
| Magnésium | 22 mg |
| Manganèse | 0,5 mg |
| Sodium | 95 mg |
| Phosphorus | 130 mg |
| Zinc | 1,6 mg |
| Chromium | 15 mg |
| Chloride | 110 mg |
| Fluor | 0,01 mg |
| Molybdène | 14 mg |
| lodine | 4 mg |
| Sélénium | 14 mg |

## Revendications

1. Composition de nutrition entérale thermostérilisée comportant par 100 mL de composition :
- 8 à 14 g de protéines totales
- 837 à 1465 kJ (200 à 350 kcal)
- les protéines totales comportant :
∘ une quantité de caséinates supérieure ou égale à 55% en poids desdites protéines totales de la composition
∘ une quantité de protéines sériques inférieure ou égale à 30% en poids desdites protéines totales de la composition
∘ une quantité de caséines inférieure ou égale à 30% en poids desdites protéines totales de la composition,
dans laquelle le ratio en poids desdits caséinates auxdites caséines étant supérieur ou égal à 70/30,
et pour laquelle la viscosité est inférieure ou égale à 800 mPa.s quand mesurée à 20°C à 100 s⁻¹ avec un viscosimètre rotatif.

2. Composition de nutrition entérale selon la revendication 1 **caractérisée en ce que** les caséinates sont choisis parmi les caséinates de sodium, les caséinates de potassium, les caséinates de calcium ou un mélange d'au moins deux parmi ceux-ci.

3. Composition de nutrition entérale selon la revendication 1 ou 2 **caractérisée en ce que** la quantité de protéines sériques est inférieure ou égale à 25% en poids des protéines totales de la composition.

4. Composition de nutrition entérale selon l'une quelconque des revendications précédentes **caractérisée en ce que** la quantité de caséine est inférieure ou égale à 25% en poids des protéines totales de la composition.

5. Composition de nutrition entérale selon l'une quelconque des revendications précédentes **caractérisée en ce que** les protéines contribuent pour 10 à 40% des apports énergétiques totaux par 100 mL de composition.

6. Composition de nutrition entérale selon la revendication 1 **caractérisée en ce qu'**elle comprend :
- 9 à 12 g de protéines totales par 100 mL de composition
- une densité énergétique d'environ 1046 kJ (250 kcal) par 100 mL de composition
- un ratio en poids de caséinates/caséines/protéines sériques d'environ 60/25/15.

7. Composition de nutrition entérale selon la revendication 6 **caractérisée en ce que** la viscosité de la composition est comprise entre 30 et 450 mPa.s quand mesurée à 20°C à 100 s⁻¹ avec un viscosimètre rotatif.

8. Utilisation d'une composition de nutrition entérale selon l'une quelconque des revendications précédentes pour nourrir une personne âgée.

9. Composition de nutrition entérale selon l'une quelconque des revendications précédentes pour son utilisation dans le traitement d'un état de dénutrition lié à la maladie.

10. Procédé de préparation d'une composition telle que définie à l'une des revendications 1 à 7 comprenant les étapes de :
- préparation d'une phase aqueuse
- ajout dans ladite préparation aqueuse d'eau, de glucides, de protéines, de minéraux et de vitamines,
- ajout de matière grasse
- refroidissement et hydratation du mélange obtenu
- stérilisation du mélange par un procédé U.H.T. dont la valeur stérilisatrice résultante est supérieure ou égale à 12, une homogénéisation étant réalisée avant ou après l'étape de stérilisation pour stabiliser l'émulsion.

11. Procédé selon la revendication 10 comprenant en outre l'ajout un ou plusieurs des composants choisis parmi des émulsifiants, qui peuvent être ajoutés en même temps que la matière grasse, l'ajout de colorants ou l'ajout d'arômes.

## Patentansprüche

1. Hitzesterilisierte enterale Ernährungszusammensetzung, pro 100 ml Zusammensetzung Folgendes umfassend:
- 8 bis 14 g Gesamtprotein
- 837 bis 1465 kJ (200 bis 350 kcal)
- Gesamtproteine, umfassend:
∘ eine Menge an Kaseinaten größer oder gleich 55 Gew.-% der Gesamtproteine der Zusammensetzung
∘ eine Menge von Serumproteinen von weniger als oder gleich 30 Gew.-% der Gesamtproteine der Zusammensetzung
∘ eine Menge an Kaseinen von weniger als oder gleich 30 Gew.-% der Gesamtproteine der Zusammensetzung, wobei das Gewichtsverhältnis der Kaseinate zu den Kaseinen größer als oder gleich 70/30 ist und wobei die Viskosität kleiner als oder gleich 800 mPa.s ist, wenn sie bei 20 °C bei 100 s⁻¹ mit einem Rotationsviskosimeter gemessen wird.

2. Enterale Ernährungszusammensetzung nach Anspruch 1, **dadurch gekennzeichnet, dass** die Kaseinate aus Natriumkaseinaten, Kaliumkaseinaten, Calciumkaseinaten oder einer Mischung von mindestens zwei davon ausgewählt sind.

3. Enterale Ernährungszusammensetzung nach Anspruch 1 oder 2, **dadurch gekennzeichnet, dass** die Menge der Serumproteine kleiner oder gleich 25 Gew.-% der Gesamtproteine der Zusammensetzung ist.

4. Enterale Ernährungszusammensetzung nach einem der vorhergehenden Ansprüche, **dadurch gekennzeichnet, dass** die Menge an Kasein weniger als oder gleich 25 Gew.-% der Gesamtproteine der Zusammensetzung beträgt.

5. Enterale Ernährungszusammensetzung nach einem der vorhergehenden Ansprüche, **dadurch gekennzeichnet, dass** Protein 10 bis 40 % der gesamten Energiezufuhr pro 100 ml der Zusammensetzung ausmacht.

6. Enterale Ernährungszusammensetzung nach Anspruch 1, **dadurch gekennzeichnet, dass** sie Folgendes umfasst:
- 9 bis 12 g Gesamtprotein pro 100 ml Zusammensetzung
- eine Energiedichte von etwa 1046 kJ (250 kcal) pro 100 ml Zusammensetzung
- ein Gewichtsverhältnis von Kaseinaten/Kaseinen/Serumproteinen von etwa 60/25/15.

7. Enterale Ernährungszusammensetzung nach Anspruch 6, **dadurch gekennzeichnet, dass** die Viskosität der Zusammensetzung zwischen 30 und 450 mPa.s liegt, wenn sie bei 20 °C bei 100 s⁻¹ mit einem Rotationsviskosimeter gemessen wird.

8. Verwendung einer enteralen Ernährungszusammensetzung nach einem der vorhergehenden Ansprüche zur Ernährung einer älteren Person.

9. Enterale Ernährungszusammensetzung nach einem der vorhergehenden Ansprüche zur Verwendung bei der Behandlung eines krankheitsbedingten unterernährten Zustands.

10. Verfahren zur Herstellung einer Zusammensetzung nach einem der Ansprüche 1 bis 7, umfassend die folgenden Schritte:
- Herstellen einer wässrigen Phase
- Zugeben von Wasser, Kohlenhydraten, Proteinen, Mineralstoffen und Vitaminen zu diesem wässrigen Präparat,
- Zugeben von Fett
- Abkühlen und Hydratisieren der entstandenen Mischung
- Sterilisieren der Mischung durch ein U.H.T.-Verfahren, dessen resultierender Sterilisationswert größer oder gleich 12 ist, wobei die Homogenisierung vor oder nach dem Sterilisationsschritt durchgeführt wird, um die Emulsion zu stabilisieren.

11. Verfahren nach Anspruch 10, ferner umfassend das Zugeben einer oder mehrerer Komponenten, ausgewählt aus Emulgatoren, die zusammen mit Fett zugegeben werden können, das Zugeben von Farbstoffen oder das Zugeben von Aromastoffen.

## Claims

1. A heat-sterilized enteral nutrition composition comprising, per 100 mL of composition:
- 8 to 14 g total in proteins
- 837 to 1465 kJ (200 to 350 kcal)
- the total of proteins comprising:
∘ a quantity of caseinates greater than or equal to 55% by weight of said total proteins of the composition
∘ a quantity of serum proteins less than or equal to 30% by weight of said total proteins of the composition
∘ a quantity of caseins less than or equal to 30% by weight of said total proteins of the composition, wherein the weight ratio of said caseinates to said caseins is greater than or equal to 70/30, and wherein the viscosity is less than or equal to 800 mPa.s when measured at 20°C at 100 s⁻¹ with a rotary viscometer.

2. The enteral nutrition composition according to claim 1, **characterized in that** the caseinates are chosen from sodium caseinates, potassium caseinates, calcium caseinates or a mixture of at least two of these.

3. The enteral nutrition composition according to claim 1 or 2, **characterized in that** the quantity of serum proteins is less than or equal to 25% by weight of the total proteins of the composition.

4. The enteral nutrition composition according to any one of the preceding claims, **characterized in that** the quantity of casein is less than or equal to 25% by weight of the total proteins of the composition.

5. The enteral nutrition composition according to any one of the preceding claims, **characterized in that** the proteins make up 10 to 40% of the total energy intake per 100 mL of the composition.

6. The enteral nutrition composition according to claim 1, **characterized in that** it comprises:
- 9 to 12 g of total protein per 100 mL of composition
- an energy density of around 1046 kJ (250 kcal) per 100 mL of composition
- a weight ratio of caseinates/caseins/serum proteins of around 60/25/15.

7. The enteral nutrition composition according to claim 6, **characterized in that** the viscosity of the composition is between 30 and 450 mPa.s when measured at 20° C at 100 s⁻¹ with a rotary viscometer.

8. A use of an enteral nutrition composition according to any one of the preceding claims for feeding an elderly person.

9. An enteral nutrition composition according to any one of the preceding claims for use in the treatment of a disease-related state of undernutrition.

10. A process for the preparation of a composition as defined in one of claims 1 to 7, comprising the steps of:
- preparation of an aqueous phase
- addition of water, carbohydrates, proteins, minerals and vitamins to said aqueous preparation,
- addition of fatty material
- cooling and hydration of the obtained mixture
- sterilization of the mixture through a UHT process. the resulting sterilizing value of which is greater than or equal to 12, a homogenization being carried out before or after the sterilization step to stabilize the emulsion.

11. The method according to claim 10, further comprising adding one or more of the components selected from emulsifiers, which may be added at the same time as the fat, adding coloring agents or adding flavors.
